# EUROPEAN PATENT SPECIFICATION

(11) **EP 2 928 534 B1**
(45) Date of publication and mention of the grant of the patent: **26.05.2021**
(21) Application number: 13808314.2
(22) Date of filing: 21.11.2013
(51) Int. Cl.: A61M 25/00, A61M 31/00, A61M 39/10

(54) **VENTURI EFFECT MIXING CATHETER APPARATUS**
VENTURI-EFFEKT-MISCHKATHETERVORRICHTUNG
APPAREIL À CATHÉTER DE MÉLANGE À EFFET VENTURI

(30) Priority: 04.12.2012 US 201261733133 P; 02.08.2013 US 201313958067
(43) Date of publication of application: 14.10.2015
(73) Proprietor: Cook Medical Technologies LLC, Bloomington, IN 47404 (US)
(72) Inventor: DUNCAN, Kate, Mooresville, IN 46158 (US)
(74) Representative: Mathys & Squire
(86) International application number: PCT/US2013/071268
(87) International publication number: WO 2014/088828

(56) References cited:
- WO-A2-2009/042621
- US-A- 5 211 627
- US-A1- 2012 035 471
- US-A1- 2012 190 980
- US-B1- 6 450 963

## Description

### Technical Field

The present invention relates generally to apparatus for delivering a solution into a body cavity, and more specifically, to a catheter apparatus for introducing a contrast media including air and saline into the uterus and fallopian tubes to create a contrast image viewable during ultrasonography.

There are a variety of techniques used for visualizing a body cavity, including the uterus and fallopian tubes. When used for diagnosing tubal patency, such techniques often rely on use of a hysteroscope or a balloon catheter to inject contrast media or dye into the uterus and fallopian tubes which may require fluoroscopy or X-ray imaging techniques for viewing and diagnosis. Such procedures are often performed in a hospital setting under some type of anesthesia.

Alternative contrast agents and methods have also been recently investigated and it has been found that air and saline provide quality contrasting capability. For example, a different type of procedure for visualizing the fallopian tubes, often referred to as Hysterosalpingocontrastsonography, or "HyCoSy", may utilize air bubbles to create a contrast image. More particularly, a mixture of air bubbles in a solution such as saline may be introduced into a body cavity to enhance visualization of the anatomical structure during ultrasound.

Reference is directed to US 6,450,963 which discloses apparatus for sonographically observing a location and condition of the body. The apparatus has a pump assembly and a catheter assembly fluidly coupled to the pump assembly. The pump assembly includes a first pump for pumping a solution of sterile saline into the catheter assembly and a second pump for pumping micro-filtered air into the catheter assembly. The catheter assembly injects the solution of sterile saline and the sterile saline mixed with micro-filtered air into a location of a body.

Reference is further directed to US 2012/0190980 which describes methods of determining the occlusion of body lumens and apparatuses for doing so. In one particular embodiment, the occlusion of the fallopian tubes by an intrafallopian contraceptive device may be confirmed by contrast enhanced ultrasonography (also known as stimulated acoustic emission hysterosalpingo-contrast sonography). In these embodiments a contrast agent containing microbubbles is used.

Reference is further directed to US 2012/0035471 which describes the creation of a contrast medium comprising detectable acoustic variations between two phases, for example, a gas and a liquid.

Reference is further directed to WO 2009/042621 which discloses a system which has an ultrasound transducer for detecting ultrasound energy reflected from inside the subject and an ultrasound contrast enhancement system for introducing microbubbles into the subject during the injection. The microbubbles increase reflectivity of one or more fluids in the subject to ultrasound energy, facilitating extravasation detection. A needle or injection line may be constructed to produce the microbubbles during the injection.

Accordingly, it is desirable to provide simplified and improved apparatus for visualizing a body cavity, including but not limited to, a catheter apparatus for generating air bubbles in saline to be introduced into the uterus and fallopian tubes for viewing and evaluating the anatomical structure thereof including diagnosing tubal patency.

### Summary

The present disclosure provides an apparatus for introducing a solution into a body cavity as defined in independent claim 1 and subsequent claims 2-13. In one example, the apparatus comprises a hub including a proximal end portion adapted for removable attachment to a solution source and a distal end portion, and a lumen having a variable diameter extending between the proximal and distal end portions. The hub further includes an airflow passage in communication with the lumen, wherein the lumen diameter is constricted between the hub proximal and distal end portions and wherein at least a portion of the constricted lumen is in communication with the airflow passage. The apparatus further comprises a catheter comprising a distal end portion and a proximal end portion and at least one lumen extending between the distal and proximal end portions. The proximal end portion of the catheter is removably attached to the hub distal end portion. The catheter may further include a positioner component or member located between the proximal and distal end portions. The positioner may include a cervical occlusion device such as an inflatable balloon or silicone bung.

In one example, the hub lumen tapers radially inwardly from the proximal end portion of the hub to the smallest diameter portion of the lumen and then tapers radially outwardly from the smallest diameter portion to the distal end portion of the hub. The geometry of the variable diameter lumen is preferably configured to create the Venturi effect, in which the hub lumen is configured to create a vacuum as a result of a reduction in fluid pressure and in increase in fluid velocity of a solution flowing through the constricted portion of the hub lumen.

In another example, a method for introducing a solution into a body cavity is described. The method may comprise the steps of providing a catheter apparatus including a hub having a proximal end portion and a distal end portion adapted for attachment to a catheter, a lumen having a constricted diameter located between the hub proximal and distal end portions and an airflow passage in communication with a portion of the hub lumen having a constricted diameter. The method may further include inserting a distal end portion of the catheter into a body cavity and flowing a solution through the hub lumen to generate a vacuum in the lumen while permitting air to flow through the airflow passage. The air may mix with the solution flowing through the hub lumen, thus resulting in an aerated mixture of air bubbles in solution which may be introduced into the body cavity through the distal end portion of the catheter.

In yet another example, a method for delivering a solution into a body cavity is described, which may include the steps of positioning the distal end of a catheter into the body cavity of a patient and injecting the solution through the lumen of a hub that is disposed outside of a patient and which lumen is in fluid communication with the catheter. The method may further include applying the Venturi effect to the solution flowing through the hub lumen to create a vacuum which causes air to be automatically drawn into the hub lumen through an air intake line to create a mixture of air bubbles in the solution within the hub lumen, and then emitting the mixture of bubbles in solution out of the distal end of the catheter and into the body cavity. In one example, the vacuum is created as a result of a fluid flow induced suction created by the solution flowing through a constricted portion of the hub lumen.

### Brief Description of the Drawings

Figure 1 is a side view of an apparatus for delivering a solution into a body cavity and a source of solution.
Figure 2 is a perspective view of a hub and a catheter for delivering a solution into a body cavity and the distal end of a syringe containing a solution adapted for communication with the hub.
Figure 3 is a cross-sectional view of an apparatus for delivering a solution into a body cavity with a solution being introduced into the proximal end thereof and flowing in a distal direction through the apparatus lumen.
Figure 4 illustrates one embodiment of the apparatus wherein the distal end of a catheter is positioned within the uterus with an inflated balloon occluding the internal cervical opening as solution is introduced into the uterus and fallopian tubes.
Figure 5 illustrates an alternative embodiment of Figure 4 wherein an occlusion device is positioned at the external cervical opening as solution is introduced into the uterus and fallopian tubes.
Figure 6 is a cross-sectional view of one embodiment of the proximal end portion of the apparatus wherein a catheter has been inserted into the distal end portion of a hub.
Figure 7 is a cross-sectional view of an alternative embodiment of the proximal end portion of the apparatus wherein a catheter having a fitting at the proximal end thereof is inserted over the tapered distal end of the hub.

### Detailed Description

Throughout this specification, the terms proximal and proximally are used to refer to a position or direction away from, or even external to a patient's body and the terms distal and distally are used to refer to a position or direction towards the patient and/or to be inserted into a patient's body orifices or cavities. The embodiments and examples described below are primarily in connection with the use of a catheter apparatus for introducing a solution, such as a mixture of air and saline into the uterus and fallopian tubes to improve visualization during ultrasound, however, the described apparatus may also be used in connection with a range of medical procedures including the introduction of various agents and/or media into other body cavities and internal orifices to enhance visualization thereof using other known methods and techniques. For example, it is also contemplated that the apparatus described herein could be used in different areas of the body where contrast media is or could be used to enhance visualization during ultrasound. This may include applications in urology and/or gastric procedures, among others, as will be appreciated by those of skill in the art.

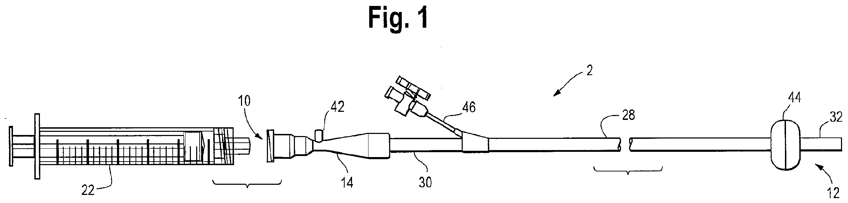

Figure 1 illustrates an example of an apparatus 2 for delivering a solution into a body cavity. The apparatus includes a proximal external manipulation section shown generally at 10 which is operated by a clinician and a distal end shown generally at 12 which is introduced into a body cavity. During a medical procedure to deliver a solution into a body cavity, at least a portion of the distal end 12 will travel through a body orifice or opening to a desired site or internal body cavity. The external manipulation section 10, which is acted upon by a user to manipulate and operate the device, remains outside of the patient throughout the procedure.

The apparatus 2 may be particularly useful in performing a non-surgical, ultrasound-based diagnostic imaging procedure for examining the anatomical structures of a body cavity. More particularly, the apparatus may be used for performing a Hysterosalpingocontrastsonography (HyCoSy) procedure. A HyCoSy procedure may include introducing a solution, such as mixture of air and saline, though a catheter and into the uterus and fallopian tubes. However, other solutions and mixtures comprising various liquids and/or gasses, foams, gels and the like, for example, may also be used as necessary or desired depending on the particular procedure being performed. The mixture of a liquid media and a gas such as air and saline causes air bubbles to be formed in the saline, which, when introduced into a body cavity, create a contrast image that can be clearly viewed by ultrasound. In one example, a physician may use a transvaginal ultrasound probe while introducing the air and saline mixture to sonographically visualize the fallopian tubes to evaluate and diagnose tubal patency in a patient.

As shown in Figure 1, the proximal external manipulation portion 10 of the device 2 includes a hub 14. The hub 14 may be a single unitary piece of molded material made by injection or insert molding or the like. Alternatively, separately molded hub pieces, such as two halves, may be attached or otherwise secured together using adhesive, bonding or known techniques to form the hub 14. The hub may be constructed or molded from various suitable materials including, but not limited to polycarbonate or other similar rigid plastics.

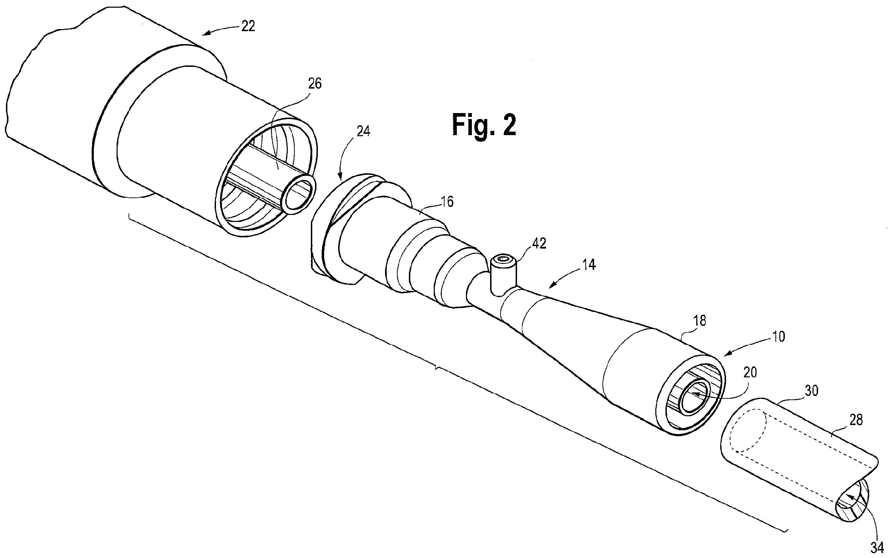

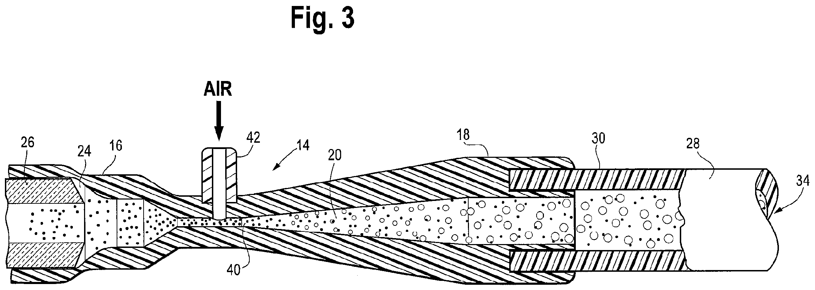

As illustrated in Figures 2 and 3, the hub 14 preferably has a proximal end 16 and a distal end 18 and a lumen 20 that extends between the proximal and distal ends. The proximal end 16 of the hub 14 is preferably configured to communicate with a solution source 22. In one example, the solution is a source of saline that may be contained in a bag, container and/or one or more syringes. The proximal end portion 16 of the hub 14 includes an opening 24, such as a female Luer lock or fitting, that is configured to receive the distal tip 26 of a saline-filled syringe 22. The tip 26 of the syringe 22 may be a correspondingly shaped male Luer that is inserted into the proximal end 16 of the hub 14 and manually held in position by the physician or alternatively, secured in this position by friction fit, snap-fit or inter-engaging structures, screw threads or the like. It is also contemplated that the proximal end portion 16 of the hub may be shaped in a variety of other ways which would allow for communication with any number or types of fluid sources 22.

As shown in Figures 1, 2 and 3, the apparatus further includes a catheter 28. The catheter 28 has a proximal end portion 30 that is removably attached or secured to the distal end portion 18 of the hub 14. The catheter 28 is preferably sufficiently flexible so that it can be advanced along a tortuous pathway for insertion into a body cavity. At least one lumen 34 extends between the proximal 30 and distal ends 32 of the catheter 28 and provides a pathway for fluid flow therethrough.

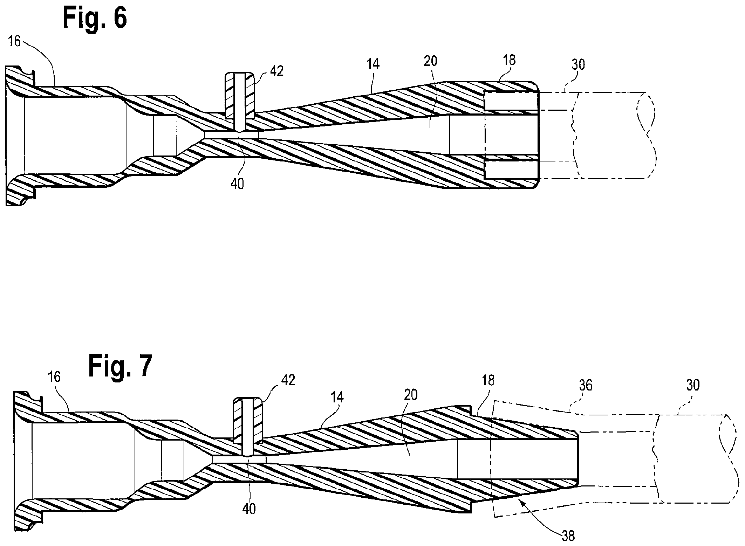

The catheter 28 may be attached or secured to the distal end 18 of the hub 14 in various ways. In one example, the proximal end 30 of the catheter 28 may include an attachment component 36, such as a female Luer fitting as shown in Figure 7. The Luer fitting 36 on the proximal end 30 of the catheter 28 may be inserted over a tapered distal end portion 38 of the hub 14. Alternatively, if the catheter 28 does not include an attachment component 36, the proximal end 30 of the catheter 28 may be inserted into the distal end 18 of the hub 14 which is preferably correspondingly shaped to receive the catheter as shown in Figures 2, 3 and 6. Of course, other known or acceptable ways of attaching the hub and catheter may also be used.

Details of one example of the inner geometry of the hub are illustrated in Figure 3. As shown there, the lumen 20 extending between the proximal 16 and distal ends 18 of the hub 14 preferably has a variable diameter. More particularly, the lumen diameter is preferably "hourglass shaped" such that it is relatively wide at the proximal end portion 16 of the hub, and tapers radially inwardly to a constricted portion 40, then tapers radially outwardly from the constricted portion 40 to the distal end portion 18 of the hub 14. Preferably, the constricted portion 14, where the diameter of the lumen is smallest or most narrow, is generally centrally located between the proximal 16 and distal 18 hub ends. Although, other configurations are also contemplated, including, but not necessarily limited to configurations where the constricted portion 40 of the lumen 20 is located closer to the hub proximal end 16 or closer to the distal end 18, or where the lumen 20 includes more than one constricted portion.

The hub 14 also preferably includes an airflow passage 42. The airflow passage 42 may be one or more apertures or openings formed in the hub 14, or, as shown in exemplary Figure 3, the airflow passage 42 is preferably in the form of an air intake tube. In one example, the air intake tube is in communication with at least a portion of the hub lumen 20. Preferably, the air intake tube 42 communicates with a portion of the lumen 20 having the most narrow or constricted diameter 40, although the air intake tube 42 may be in communication with any one or more portions of the hub lumen 20. In addition, the air intake intake tube 42 may also include a filter (not shown). The filter may be located within or inserted into a portion of the tube 42 or, alternatively, the filter may be capped over the tip of the tube and secured in place by friction fit, bonding, adhesive or the like. The filter may be any number of types and sizes, including, but not limited to 0.1 micron to 0.45 micron, and preferably, a 0.2 to 0.3 micron pore filter, for example, to substantially eliminate or prevent contaminants from entering through the air intake 42 and into the hub lumen 20 (and stream of solution flowing therethrough). Although, the type and size of the filter may vary and selection thereof may depend on various factors such as the desired filtration level.

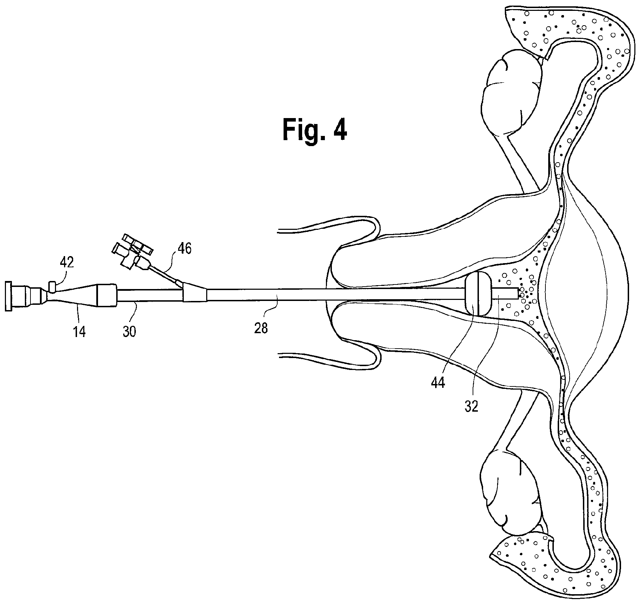

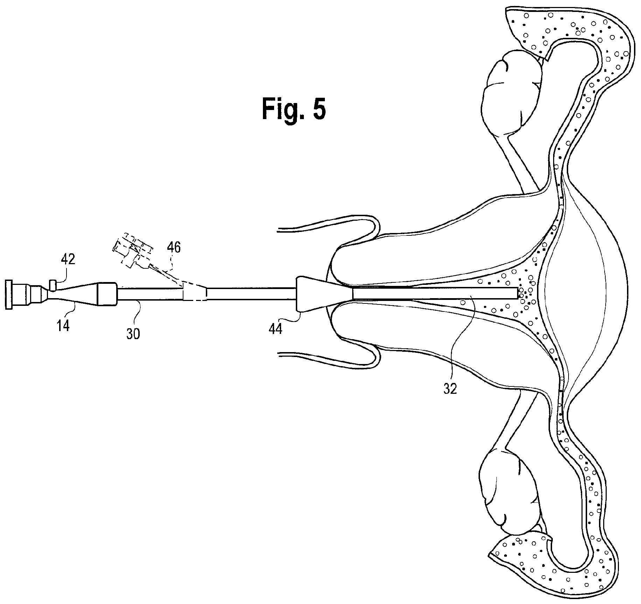

Turning back now to Figure 3, the internal geometry of the hub 14 is preferably configured to create a vacuum when fluid passes through the hub lumen 20. More particularly, the configuration of the hub preferably creates the "Venturi effect" in which a vacuum suctions or pulls air into a stream of solution as it flows though the hub lumen 20. This "Venturi effect" or vacuum is the result of a reduction in fluid pressure and in increase in fluid velocity of the solution flowing through the constricted portion 40 of the hub lumen 20. Thus, as shown in Figure 3, as a solution such as saline is introduced into the proximal end 16 of the hub, it flows through the lumen 20 towards the distal end portion 18 of the hub 14. As the saline passes through the constricted portion 40, the vacuum created in the lumen 20 draws air through the air intake tube 42. As the stream of saline continues to pass in a distal direction underneath and past the air intake tube 42, the air and saline mix within the lumen 20 to form an aerated mixture of small "microbubbles" in the saline before the mixture enters the catheter 28. The mixture may then flow through the catheter 28 and be expelled from the distal end 32 thereof into the patient's uterus and fallopian tubes as shown in Figures 4 and 5 and as described in further detail below. The size of the bubbles created when the air mixes with the saline may be controlled by altering the inner diameter of the air intake tube 42. For example, the smaller the inner diameter of the air intake tube, the smaller the bubbles that will be created. Conversely, increasing the inner diameter of the air intake tube will create larger bubbles in the saline.

As shown in Figures 1, 4 and 5, a positioner and/or occlusion member 44 may be mounted to at least a portion of the catheter 28, and preferably, near the distal end portion 32 of the catheter. In one example, the occlusion member 44 may be a component that is mounted to an external surface of the catheter and which extends radially outwardly therefrom. This may include a silicone bung or disc that is bonded or secured by friction fit to the external surface of the catheter, such that when the distal end portion of the catheter is inserted through the cervix, the silicone bung may abut the external cervical opening as shown in Figure 5.

Alternatively, the occlusion member 44 may be an inflatable balloon as Figures 1 and 4 illustrate. In such case, the catheter 28 may include an additional inflation lumen to allow for balloon inflation and deflation. For example, the inflation lumen may have a side port open only to the balloon but which is closed on the distal end of the catheter. In this way, only one of the two catheter lumens- e.g. the "fluid lumen" serves to introduce fluid into the uterus, while the second "inflation lumen" will only communicate with the balloon but not with a body cavity.

As also shown in Figures 1 and 4, an inflation line or tube 46 that may include a stopcock or check-valve, may also be provided. The inflation line 46 may be integrally molded as part of the hub, or alternatively, removably attachable to the hub and/or to the proximal end portion 30 of the catheter. The inflation line 46 preferably communicates with the inflation lumen of the catheter to allow for manipulation of the balloon as necessary, including inflation and deflation thereof. In use, the distal end portion 32 of the catheter 28, with the balloon in a deflated state carried thereon, may be inserted through the cervix and into the uterus. Once the deflated balloon has passed through the cervix, it may be inflated so that it abuts the internal cervical opening as shown in Figure 4 to hold the catheter in position while occluding the cervical opening.

As best illustrated in Figures 4 and 5, when a solution, such as a mixture of air and saline, is introduced into the uterus, it will fill the uterus and fallopian tubes (i.e. traveling the path of least resistance) while the occlusion member 44 substantially prevents the solution from flowing backwards and leaking out of the uterine cavity though the cervical opening. The occlusion member 44 also preferably serves as a positioner, in that it helps a physician to locate the relative position of the distal end 32 of the catheter 28 during insertion though the cervix and into the uterus, and also holds the catheter 28 in a particular position uterus after a physician has introduced it into a desired location within the uterus.

In one example, the dimensions of the apparatus may be as follows, although other dimensions of the particular components and/or combination thereof may be altered as necessary or desired. In one example, the catheter 28 may have a working length of between about 25 cm and 30 cm long, measured from the distal end 18 of the hub 14. The outer diameter of the catheter 28 may be between approximately 7-9 Fr (e.g. 2.3 mm to 3.0 mm). The inner diameter of the catheter 28 may be dependent on whether an inflatable occlusion balloon 44 is present or not. If present, the inner diameter of the catheter lumen 34 may be smaller in order to accommodate both a fluid lumen and an inflation lumen. If a balloon 44 is present, the balloon may be located about 10 mm from the distal tip 18 of the catheter 28 and approximately 7 mm long. The inflated outer diameter of the balloon may be between about 12mm and 15 mm.

The above-described apparatus 2 may be operated in accordance with the following exemplary methods. First, a patient is preferably placed in the dorsal lithotomy position to allow a physician to insert the distal end 32 of the catheter 28 through the patient's cervix and into the uterine cavity. When the catheter is in the desired position, the physician may occlude the cervix with the occlusion member 44 and/or hold the catheter 28 in the desired position within the uterus. This may be accomplished by inflating a balloon 44 inside the uterus through the inflation lumen to occlude the internal cervical opening as Figure 4 best illustrates. An alternative occlusion member, such as a silicone bung shown in Figure 5 that extends radially outwardly from the external catheter surface as described above, may also be used alone or in combination with the balloon to occlude the external cervical opening and/or assist in holding the catheter 28 in a desired position within the uterus.

A source of a solution 22, such as a saline-filled syringe, may be placed in communication with the proximal end 16 of the hub 14. In one example, the physician may insert the syringe tip 26 into a Luer fitting 24 located at the proximal end 16 of the hub 14, and using one hand, inject the saline into the hub lumen 20. As mentioned briefly above, the internal geometry of the hub 14, and in particular the configuration of the hub lumen 20, preferably creates the Venturi effect. In other words, the hub lumen 20 is shaped such that it allows air to be drawn into the lumen 20 through the airflow passage 42 as a result of the vacuum created by the saline solution flowing through the lumen constricted portion 40. As the air and saline mix, small bubbles are automatically created, without the need for providing a separately contained source of air (e.g. an air pump or air-filled syringe) and without the need for the physician to separately inject air into the system or manually prepare a mixture of saline and air prior to performing a particular procedure. As shown in Figures 3-5, the mixture of bubbles in saline flow through the catheter 28, and, upon exiting the distal end 32 of the catheter, fill the uterus and fallopian tubes.

The physician will be able inspect the patient's anatomy as the mixture of bubbles in saline is introduced into the uterus and flows through the fallopian tubes. For example, if desired, the physician may use the other "free" hand (i.e. the hand that is not being used to operate the catheter apparatus 2) to operate an ultrasound probe or wand, or alternatively, another individual may aid in operating the ultrasound equipment. Preferably, a transvaginal ultrasound probe can be guided into the patient's vagina. The contrast image crated by the air bubbles in saline will allow the physician to sonographically visualize the fallopian tubes and provide an opportunity for diagnosing tubal patency, for example.

Throughout this specification, unless the context requires otherwise, the words "comprise" and "include" and variations such as "comprising" and "including" will be understood to imply the inclusion of an item or group of items, but not the exclusion of any other item or group items.

While various embodiments of the invention have been described, it will be apparent to those of ordinary skill in the art that many more embodiments and implementations are possible within the scope of the invention. Furthermore, although various indications have been given as to the scope of this invention, the invention is not limited to any one of these but may reside in two or more of these combined together. Accordingly, the invention is not to be restricted except in light of the attached claims and their equivalents.

## Claims

1. An apparatus for introducing a solution into a body cavity comprising:
a hub (14) comprising
a proximal end portion (16) adapted for removable attachment to a solution source (22) and a distal end portion (18),
a lumen (20) having a variable diameter and extending between the hub proximal and distal end portions,
an airflow passage (42) in communication with the lumen,
wherein the lumen diameter is constricted between the hub proximal and distal end portions and wherein at least a portion of the constricted lumen is in communication with the airflow passage;
a catheter (28) comprising a distal end portion and a proximal end portion and at least one lumen extending between the distal and proximal end portions, the proximal end portion of the catheter removably attached to the hub distal end portion.

2. The apparatus of claim 1 wherein the hub comprises an integral piece of molded material.

3. The apparatus of claim 1 wherein the hub comprises multiple pieces of molded material secured together.

4. The apparatus of claim 1 wherein the airflow passage further comprises a filter.

5. The apparatus of claim 1 further comprising a positioner member located between the catheter proximal and distal end portions.

6. The apparatus of claim 5 wherein the positioner comprises a cervical occlusion device secured to an outer surface of the catheter.

7. The apparatus of claim 1 wherein the catheter comprises at least two lumens.

8. The apparatus of claim 7 wherein at least one of the two lumens comprises a balloon inflation lumen.

9. The apparatus of claim 1 wherein the hub further comprises a Luer fitting at the proximal end portion of the hub adapted for communication with the solution source.

10. The apparatus of claim 1 wherein the portion of the variable diameter lumen having the smallest diameter is substantially centrally located between the hub proximal and distal ends.

11. The apparatus of claim 10 wherein the lumen tapers radially inwardly from the proximal end portion of the hub to the smallest diameter portion and then tapers radially outwardly from the smallest diameter portion to the distal end portion of the hub.

12. The apparatus of claim 1 wherein the hub lumen is configured to create a vacuum as a result of a reduction in fluid pressure and in increase in fluid velocity of the solution flowing through the constricted portion of the hub lumen.

13. The apparatus of claim 12 wherein the airflow passage is configured to allow air to be drawn therethrough and into a stream of solution flowing through the hub lumen as a result of the vacuum created.

## Patentansprüche

1. Vorrichtung zum Einbringen einer Lösung in eine Körperhöhle, umfassend:
einen Ansatz (14), umfassend:
einen proximalen Endabschnitt (16), der zur lösbaren Befestigung an einer Lösungsquelle (22) vorgesehen ist, und einen distalen Endabschnitt (18),
ein Lumen (20) mit einem variablen Durchmesser, und das sich zwischen dem proximalen und dem distalen Endabschnitt des Ansatzes erstreckt,
einen Luftströmungsdurchgang (42) in Kommunikation mit dem Lumen,
wobei der Lumendurchmesser zwischen dem proximalen und dem distalen Endabschnitt des Ansatzes verengt ist, und wobei mindestens ein Abschnitt des verengten Lumens in Kommunikation mit dem Luftströmungsdurchgang ist;
einen Katheter (28), der einen distalen Endabschnitt und einen proximalen Endabschnitt und mindestens ein Lumen umfasst, das sich zwischen dem distalen und dem proximalen Endabschnitt erstreckt, wobei der proximale Endabschnitt des Katheters entfernbar an dem distalen Endabschnitt des Ansatzes befestigt ist.

2. Vorrichtung nach Anspruch 1, wobei der Ansatz ein integrales Teil aus geformtem Material umfasst.

3. Vorrichtung nach Anspruch 1, wobei der Ansatz mehrere Teile aus geformtem Material umfasst, die aneinander gesichert sind.

4. Vorrichtung nach Anspruch 1, wobei der Luftströmungsdurchgang des Weiteren einen Filter umfasst.

5. Vorrichtung nach Anspruch 1, das des Weiteren ein Positioniererelement umfasst, das sich zwischen dem proximalen und dem distalen Endabschnitt des Katheters befindet.

6. Vorrichtung nach Anspruch 5, wobei der Positionierer eine zervikale Okklusionsvorrichtung umfasst, die an einer Außenseite des Katheters gesichert ist.

7. Vorrichtung nach Anspruch 1, wobei der Katheter mindestens zwei Lumina umfasst.

8. Vorrichtung nach Anspruch 7, wobei mindestens eines der beiden Lumina ein Ballonbefüllungslumen umfasst.

9. Vorrichtung nach Anspruch 1, wobei der Ansatz des Weiteren ein Luer-Anschlussstück an dem proximalen Endabschnitt des Ansatzes umfasst, das zur Kommunikation mit der Lösungsquelle vorgesehen ist.

10. Vorrichtung nach Anspruch 1, wobei der Abschnitt des Lumens mit variablem Durchmesser, der den kleinsten Durchmesser aufweist, sich im Wesentlichen zentral zwischen dem proximalen und distalen Ende des Ansatzes befindet.

11. Vorrichtung nach Anspruch 10, wobei das Lumen sich radial einwärts von dem proximalen Endabschnitt des Ansatzes zu dem Abschnitt mit kleinstem Durchmesser abschrägt, und sich dann radial auswärts von dem Abschnitt mit dem kleinsten Durchmesser auswärts zu dem distalen Endabschnitt des Ansatzes abschrägt.

12. Vorrichtung nach Anspruch 1, wobei das Lumen des Ansatzes ausgestaltet ist, um infolge einer Reduktion des Fluiddrucks und eines Anstiegs der Fluidgeschwindigkeit der Lösung, die durch den verengten Abschnitt des Lumens des Ansatzes strömt, ein Vakuum zu erzeugen.

13. Vorrichtung nach Anspruch 12, wobei der Luftströmungsdurchgang ausgestaltet ist, um zuzulassen, dass infolge des erzeugten Vakuums Luft hindurch und in einen Strom der Lösung gezogen wird, die durch das Lumen des Ansatzes strömt.

## Revendications

1. Appareil pour introduire une solution dans une cavité corporelle, comprenant :
un moyeu (14) comprenant :
une portion d'extrémité proximale (16) prévue pour être attachée de manière amovible à une source de solution (22) et une portion d'extrémité distale (18),
une lumière (20) de diamètre variable, s'étendant entre les portions d'extrémité proximale et distale du moyeu,
un passage d'air (42) en communication avec la lumière,
le diamètre de la lumière étant rétréci entre les portions d'extrémité proximale et distale du moyeu et au moins une portion de la lumière rétrécie étant en communication avec le passage d'air ;
un cathéter (28) comprenant une portion d'extrémité distale et une portion d'extrémité proximale et au moins une lumière s'étendant entre les portions d'extrémité distale et proximale, la portion d'extrémité proximale du cathéter étant attachée de manière amovible à la portion d'extrémité distale du moyeu.

2. Appareil selon la revendication 1, dans lequel le moyeu comprend un morceau intégral de matériau moulé.

3. Appareil selon la revendication 1, dans lequel le moyeu comprend plusieurs morceaux de matériau moulé fixés les uns aux autres.

4. Appareil selon la revendication 1, dans lequel le passage d'air comprend en outre un filtre.

5. Appareil selon la revendication 1, comprenant en outre un organe de positionnement situé entre les portions d'extrémité proximale et distale du cathéter.

6. Appareil selon la revendication 5, dans lequel l'organe de positionnement comprend un dispositif d'occlusion cervicale fixé à une surface extérieure du cathéter.

7. Appareil selon la revendication 1, dans lequel le cathéter comprend au moins deux lumières.

8. Appareil selon la revendication 7, dans lequel au moins l'une des deux lumières comprend une lumière de gonflage de ballonnet.

9. Appareil selon la revendication 1, dans lequel le moyeu comprend en outre un raccord de Luer au niveau de la portion d'extrémité proximale du moyeu, prévu pour communiquer avec la source de solution.

10. Appareil selon la revendication 1, dans lequel la portion de la lumière de diamètre variable ayant le plus petit diamètre est située sensiblement centralement entre les extrémités proximale et distale du moyeu.

11. Appareil selon la revendication 10, dans lequel la lumière est effilée radialement vers l'intérieur depuis la portion d'extrémité proximale du moyeu jusqu'à la portion de plus petit diamètre puis est effilée radialement vers l'extérieur depuis la portion de plus petit diamètre jusqu'à la portion d'extrémité distale du moyeu.

12. Appareil selon la revendication 1, dans lequel la lumière du moyeu est configurée pour créer un vide résultant d'une réduction de pression de fluide et d'une augmentation de la vitesse de fluide de la solution s'écoulant à travers la portion rétrécie de la lumière du moyeu.

13. Appareil selon la revendication 12, dans lequel le passage d'air est configuré pour permettre à l'air d'être aspiré à travers lui et jusque dans un écoulement de solution s'écoulant à travers la lumière du moyeu sous l'effet du vide créé.
